# EUROPEAN PATENT APPLICATION

(11) **EP 4 809 907 A1**
(43) Date of publication of application: **23.09.2026**
(21) Application number: 25223026.3
(22) Date of filing: 12.12.2025
(51) Int. Cl.: A61B 5/00

(54) **BONE DENSITY MEASUREMENT SYSTEM**

(30) Priority: 21.03.2025 CN 202510339919
(71) Applicant: Cheung, Raymond Siu Wah, Kowloon (HK)
(72) Inventor: CHEUNG, Raymond Siu Wah, Kowloon (HK); YAU, Shui Wah Ivan, Kowloon (HK)
(74) Representative: Cabinet Chaillot

(57) **Abstract**

The present application relates to a bone density measurement system. In one embodiment, the system comprises a first computing device (1007) including a first processor (10071) and a first non-volatile storage medium (10072). The storage medium stores a first computer program, and the computing device is configured to issue a first instruction (1st_instru) in response to receiving input information. The system further comprises at least two signal transceivers, including a second transceiver (1005) and a third transceiver (1006). In response to receiving at least two feedback signals from the second and third transceivers, the first processor executes the first computer program and outputs an operation result (oper_result).

## Description

### FIELD OF INVENTION

The present invention relates to a bone density measurement system. The system can be applied to animals other than humans and, in particular, can be used on the human body for non-diagnostic purposes. When applied to humans, it provides the public with preliminary (non-diagnostic) indicators of bone density.

### BACKGROUND

In both animal physiology research and human orthopedic medicine field, there exists a prevalent need to measure bone density in animals/humans for medical research or for diagnosing diseases in patients.

Current bone density measurement devices and systems can be primarily categorized into two types based on their measurement methods: Radiation-based bone density measurement devices and Ultrasound-based bone density measurement devices.

### 1. Radiation-Based Bone Density Measurement Devices

A representative example of radiation-based bone density measurement devices is computed tomography (CT), which uses X-ray beams to perform tomographic scanning of animals/human bodies. The advantage of this type of bone density measurement device lies in its precise measurement results, clearly displaying animal soft tissues and bones through differential X-ray absorption by different organs and tissues. Therefore, in clinical medicine and animal physiology research fields, CT-based bone density measurement devices are widely used to measure bone density in humans/animals. Particularly in animal physiology research, it is common practice to measure bone density in mice through castration, application of various environmental variables to the mice, and using CT scanning, to study the degree of changes in animal bone density occurring with variations in different environmental factors.

Since this type of bone density measurement device requires the use of radiation, its production, purchase and use are typically subject to regulatory control by various national governments. For example, according to the China official announcement document (Document Number: 2017 No. 66) issued by the Ministry of Environmental Protection of the People's Republic of China and the National Health and Family Planning Commission on 5 December 2017, radiation-based bone density measurement devices are classified as at least Category III radiation devices and are not exempt from regulatory control. Therefore, radiation-based bone density measurement devices generally must be installed and operated at government-supervised institutions/facilities, and ordinary consumers cannot freely purchase such radiation-based devices. Furthermore, it should be noted that since specific devices are required to generate radiation and ensure that humans/animals are not adversely affected by the radiation, radiation-based bone density measurement devices typically have large physical dimensions. Due to the difficulty in further reducing their size, in animal physiology research, generally only smaller animals (e.g., mice) are typically used for studies on the correlation between bone density and environmental factors, enabling these animals to be irradiated by the rays emitted by ray-based bone density measurement devices.

### 2. Ultrasound-Based Bone Density Measurement Devices

Since ray-based bone density measurement devices are subject to strict regulation by governments in various countries/regions, and since radioactive radiation can indeed cause harm to the human body, commercial and civilian bone density measurement devices generally employ ultrasound-based bone density measurement devices. The working principle of such devices is as follows: as ultrasound waves pass through human tissues, different tissues (e.g., bones, soft tissues, etc.) are identified based on variations in the transmitted ultrasound energy. Compared to ray-based bone density measurement devices, ultrasound-based devices are not subject to stringent government oversight. Therefore, household bone density measurement devices are typically ultrasound-based.

Although diagnostic ultrasonic bone density measurement devices are not subject to government control, their production is still subject to strict governmental regulation. For example, according to the China standard GB10152-2009 (issued by the General Administration of Quality Supervision, Inspection and Quarantine of the People's Republic of China and the Standardization Administration of China on November 15, 2009), ultrasonic bone density measurement devices shall at least comply with the requirements of the China standard GB9706.1 (issued by the State Administration for Market Regulation and the Standardization Administration of China on 9 April 2020). In addition, China standard GB9706.205 (issued by the State Administration for Market Regulation and the Standardization Administration of China on 23 July 2020) also provides specific and strict provisions for the production and manufacturing of ultrasonic diagnostic/medical equipment. For example, according to Sections 201.12.4.4.101 and 201.12.4.4.103 of the GB9706.205 standard, ultrasonic diagnostic/medical equipment must be equipped with output control devices and timers that comply with mandatory specifications. Therefore, although ultrasonic bone density measurement devices have been widely used in the civilian and commercial medical markets, such devices are generally large in size and are operated by specialized personnel in dedicated locations. Even though ultrasonic measurement devices for home use have been developed, due to the constraints of national mandatory standards, the technical complexity and resulting cost of these devices remain high. Moreover, it is worth noting that ultrasonic bone density measurement devices are currently rarely applied in the field of animal physiology. This is because ultrasonic measurement devices require the ultrasonic transducer probe to be moved along a specific region of the animal/human body. Due to the size of animals and the fact that animals generally have more body hair than humans, it is difficult to apply existing ultrasonic measurement devices in the field of animal physiology.

In addition, some researchers have proposed using mechanical vibration to study the state of bone density, such states may include, for example: the stiffness of fracture healing, or the percentage of fracture healing. However, this type of research method cannot directly measure bone density, and the premise under which these research methods are valid is that the subject's bone has been injured. In real life, not all individuals have suffered orthopedic trauma; some patients who require bone density measurement may only need it due to osteoporosis-which cannot be regarded as orthopedic trauma.

Therefore, there is a need to provide a novel, user-friendly bone density measurement system with low complexity, suitable for ordinary households and general animal physiology researchers. This system should be capable of measuring the state of bone density for non-diagnostic purposes, thereby offering preliminary indicative information about bone density to general users and animal physiology researchers.

### SUMMARY OF INVENTION

The present disclosure provides several embodiments, which can solve at least one or more of the aforementioned technical problems.

The present application discloses a bone density measurement system that can be used for non-diagnostic purposes in non-human animals, comprising: a first computing device having a first processor and a first non-volatile storage medium, wherein the first non-volatile storage medium stores a first computer program, and the first computing device is further capable of issuing a first instruction in response to receiving input information; at least two signal transceivers, the at least two signal transceivers including a second transceiver and a third transceiver; wherein, in response to receiving at least two feedback signals from the second transceiver and the third transceiver, the first processor is configured to invoke the first computer program and outputs an operation result of the first computer program.

Preferably, the second transceiver and the third transceiver are respectively located at a second position and a third position that are different from each other, and at a first position different from the second position and the third position, there exists a first signal.

Preferably, the bone density measurement system further comprises a first transceiver, wherein, in response to receiving the input information, the first computing device issues the first instruction, causing the first transceiver to emit the first signal.

Preferably, the bone density measurement system further has the following features: the first transceiver, the second transceiver, and the third transceiver are respectively located at a first position, a second position, and a third position that are different from each other.

Preferably, the bone density measurement system further has the following features: the first transceiver at least includes a first signal transceiver device, a second processor, a second non-volatile storage medium, and a vibration generator, wherein the second non-volatile storage medium stores a second computer program; the second transceiver at least includes a second signal transceiver device, a third processor, a third non-volatile storage medium, and a first vibration sensor, wherein the third non-volatile storage medium stores a third computer program; the third transceiver at least includes a third signal transceiver device, a fourth processor, a fourth non-volatile storage medium, and a second vibration sensor, wherein the fourth non-volatile storage medium stores a fourth computer program.

Preferably, the bone density measurement system further has the following features: in response to the first signal transceiver device receiving the first instruction, the second processor invokes the second computer program, causing the vibration generator to emit the first signal; in response to the second signal transceiver device receiving the first signal, the first vibration sensor converts the first signal into an electrical signal for processing by the third processor, and the third processor invokes the third computer program, causing the second signal transceiver device to emit a first feedback signal; in response to the third signal transceiver device receiving the first signal, the second vibration sensor converts the first signal into an electrical signal for processing by the fourth processor, and the fourth processor invokes the fourth computer program, causing the third signal transceiver device to emit a second feedback signal; in response to the first computing device receiving the first feedback signal and the second feedback signal, the first processor invokes the first computer program to perform operations on the first feedback signal and the second feedback signal and obtain an operation result of the first computer program; wherein the at least two feedback signals include the first feedback signal and the second feedback signal.

Preferably, the bone density measurement system further has the following features: wherein the performing operations on the first feedback signal and the second feedback signal includes: performing a subtraction operation and a correlation operation on the first feedback signal and the second feedback signal to obtain a third feedback signal; obtaining information about bone density based on the third feedback signal; wherein the correlation operation may include: normalizing each feedback signal according to a difference in amplitude (or power) of the first signal (optional), and further dividing a difference between the first feedback signal and the second feedback signal by an amplitude of the first feedback signal.

Preferably, the bone density measurement system further has the following features: wherein, obtaining information about bone density based on the third feedback signal includes: performing principal component analysis on the third feedback signal to obtain at least several frequency points associated with the third feedback signal, characteristic values corresponding to the several frequency points, and several observation values corresponding to the several frequency points; inputting the several frequency points, the characteristic values, and the several observation values into a first database, wherein the first database is capable of performing matching operations between the several frequency points and reference data stored internally in the first database and returning/outputting several pieces of information about bone density.

Preferably, the bone density measurement system further comprises the following features: wherein performing the matching operation and returning a plurality of pieces of information about bone density comprises: for each frequency point among the plurality of frequency points, searching within the first database for data corresponding to the frequency point, selecting the data closest to the observation value of the frequency point as the bone density, and outputting the bone density; or for each frequency point among the plurality of frequency points, searching within the first database for data corresponding to the frequency point, selecting a plurality of data closest to the observation value of the frequency point, and applying an interpolation/extrapolation algorithm to the plurality of data to obtain an interpolation/extrapolation result, using the interpolation/extrapolation result as the bone density, and outputting the bone density.

Preferably, the bone density measurement system further has the following features: wherein, the operation result of the first computer program includes the bone density and/or a first comprehensive bone density obtained for each frequency point among the several frequency points, the first comprehensive bone density being obtained by performing a weighting operation on the bone densities corresponding to each frequency point among the several frequency points, the weighting operation including: according to the different proportions of the characteristic values corresponding to each frequency point in the principal component analysis among the characteristic values of all frequency points, multiplying the bone density corresponding to each frequency point by the weight corresponding to the characteristic value corresponding to each frequency point and summing the results.

Preferably, the bone density measurement system further has the following features: wherein, obtaining information about bone density based on the third feedback signal includes: inputting the third feedback signal into a second database, wherein the second database is capable of, according to the least squares criterion, matching the reference data stored therein one by one with the third feedback signal, and selecting the reference data with the lowest mean square error with the third feedback signal as bone density, and outputting the bone density as the operation result of the first computer program.

Preferably, the bone density measurement system further has the following features: wherein, the first signal is a mechanical vibration, and it is in the form of a pulse vibration (or an impulse function) in the time domain, its frequency range is 2000Hz-7000Hz, and the number of frequency points is one of the following: 16, 32, 64, 128, 256, 512, 1024, 2048.

Preferably, the bone density measurement system further has the following features: the number of samples of the first signal in the time domain should be at least half of the number of frequency points of the third feedback signal (preferably equal to the number of frequency points), and the sampling frequency of the first signal in the time domain should be greater than or equal to twice the sampling frequency of the third feedback signal, and particularly preferably the sampling frequency of the first signal in the time domain should be greater than or equal to five times the sampling frequency of the third feedback signal.

Preferably, the bone density measurement system further has the following features: the first transceiver is absent (or in other words, the first transceiver does not exist in the aforementioned embodiments), and the first signal is a signal whose amplitude is within a certain range.

Preferably, the bone density measurement system further has the following features: the first signal is generated in the following manner: in response to the input information including an indication of manual mode, the first computing device receives several inputs from the first vibration sensor of the second transceiver integrated in the bone density measurement system, determines an amplitude range based on the several inputs; the first computing device issues the first instruction; in response to the energy of the first feedback signal in the time domain not falling within the amplitude range, the first feedback signal and the corresponding second feedback signal are ignored.

Preferably, the bone density measurement system further has the following features: wherein, the first instruction, the first feedback signal and the second feedback signal are signals in any one of the following forms: radio, laser, visible light, Bluetooth, infrared light.

Preferably, the bone density measurement system further has the following features: wherein, the first computing device further comprises a transceiver device, the transceiver device being at least capable of receiving signals corresponding to the first instruction and the at least two feedback signals.

Preferably, the bone density measurement system further has the following features: wherein, the first database and the second database are the same database.

Preferably, the bone density measurement system further has the following features: wherein, one or both of the first database and the second database are located in the following computing devices: the first computing device and/or a cloud server.

Preferably, the bone density measurement system further has the following features: the first position is the hock or tarsal bone of a quadrupedal ungulate animal, the second position is a position substantially at the same horizontal level as the first position, the third position is the fetlock or pastern or coronary or hoof bone of the quadrupedal ungulate animal. Particularly preferably, the second position and the third position are located on the same bone.

Preferably, the bone density measurement system further has the following features: the first position is the kneecap of an ape animal, the second position is the medial malleolus of the tibia (or middle of femoral condyle) of the ape animal, the third position is the medial malleolus bone of the ape animal.

### BRIEF DESCRIPTION OF DRAWINGS

Figure 1a is a schematic diagram of a prior art ultrasound bone densitometer.
Figure 1b and Figure 1c are schematic diagrams of prior art CT bone densitometers.
Figure 2 is a schematic diagram of the bone density measurement system disclosed in the embodiments of the present application.
Figures 3a-3d respectively show structural schematic diagrams of various computing devices according to the embodiments of the present application.
Figures 4a-4c respectively show exemplary arrangement methods for the positions of various computing devices in the bone density measurement system of the embodiments of the present application.
Figures 5a-5b respectively show a schematic diagram of the principle of using acoustic equipment to detect fiberboard in the prior art and a schematic diagram of the principle of using acoustic devices to measure the bone density of a test object in the embodiments of the present application.
Figure 6 shows a frequency response curve diagram of a certain type of animal body with specific biological attributes and different bone densities.
Figures 7a-7j show methods, processes, and steps that can achieve the technical objectives of the various embodiments of the present application for non-diagnostic purposes and applicable to non-human animal bodies.

### Reference Numerals List

Hock 0001; Tarsal bone 0002; Fetlock 0003; Pastern 0004; Coronary bone 0005; Hoof bone 0006; Knee joint 0007; Tibia 0008; Kneecap 0101; Middle of femoral condyle 0102; Medial malleolus of tibia 0103; Tibia 0104; Medial malleolus bone 0105; Lateral malleolus bone 0106; Ultrasound bone densitometer 1001; Ultrasound probe 1002; CT bone densitometer 1003; First transceiver 1004; First signal transceiver device 10041; Second processor 10042; Second non-volatile storage medium 10043; Vibration generator 10044; Second transceiver 1005; Second signal transceiver device 10051; Third processor 10052; Third non-volatile storage medium 10053; First vibration sensor 10054; Third transceiver 1006; Third signal transceiver device 10061; Fourth processor 10062; Fourth non-volatile storage medium 10063; Second vibration sensor 10064; First computing device 1007; First processor 10071; First non-volatile storage medium 10072; First instruction 1st_instru; First signal 1st_sig; First feedback signal 1st_feed; Second feedback signal 2nd_feed; General feedback signal gen_feed; Operation result oper_result; Cloud clo; Bus bus; First/second/third/fourth microphone Mic.1/Mic.2/Mic.3/Mic.4; Loudspeaker Loud.; Test sample TEST_Sa; Impedance tube IM_TU.

Since the skeletal structures of ape-like animals and quadrupedal animals are partially similar, for clarity in describing the embodiments of the present application, the applicant declares that in the disclosed embodiments of this application: Features with the prefix "00" represent body parts of quadrupedal ungulate animals; and Features with the prefix "01" represent parts of ape-like animals (including humans).

### DETAILED DESCRIPTION

To facilitate the description of the embodiments of the present application, the applicant defines: "medial" refers to, when a (quadrupedal) animal is standing, taking its perspective as the reference viewpoint, the relative position between its left hind limb and right hind limb as "medial"; for ape-like animals (including humans), it refers to the position relative to the left foot and right foot as "medial" (or the position of the medial malleolus as "medial"); "lateral" is defined opposite to "medial", referring to, when a (quadrupedal) animal is standing, taking its perspective as the reference viewpoint, the direction away from its left hind limb and/or right hind limb as "lateral"; for ape-like animals (including humans), it refers to the position opposite to the "medial" position of the left foot and right foot as "lateral" (or the position of the lateral malleolus as "lateral"). In the embodiments of this application, "superior", "inferior", "left" and "right" refer to directions based on the animal's perspective when standing.

It should be declared that in the embodiments disclosed in this application, it is assumed that the measured animal/human bones have not suffered orthopedic-level trauma (for example, fractures, previous bone breaks, etc.). If the measured animal/human bones have indeed experienced trauma, those skilled in the art may adjust the technical solutions disclosed in the embodiments of this application and utilize existing models of healing progression for traumatized bones disclosed in prior art to perform the measurement, as disclosed in patent document US6024711A.

Bone densitometers (bone density measurement devices) are known to those skilled in the art. Typical bone densitometers include ultrasound bone densitometers and CT bone densitometers. These bone densitometers generally require operation by professional medical personnel.

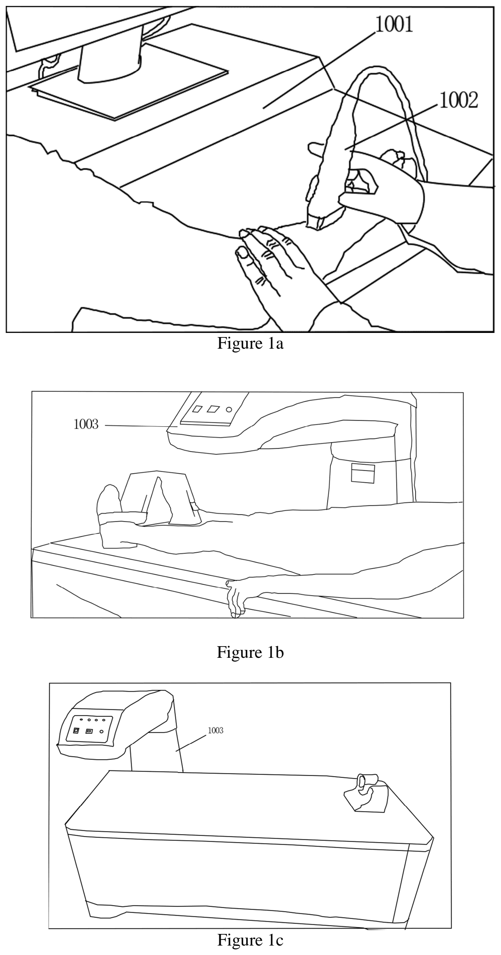

For example, referring to Figure 1a, it shows medical personnel using an ultrasound bone densitometer 1001 to measure a patient's bone density. The specific measurement method is: medical personnel first apply lubricant to the patient's arm, then use an ultrasound probe 1002 to move it back and forth along the lubricated area. During this process, the ultrasound probe 1002 continuously emits ultrasound waves toward the patient's right arm in Figure 1a. After passing through the patient's right arm, these ultrasound waves produce different acoustic field distributions. Through calculation by the computing device of the ultrasound bone densitometer 1001, the user's bone density can be inferred.

For example, referring to Figure 1b, it shows the process of applying a CT bone densitometer to measure a user/patient's bone density. The patient lies flat on the platform of the CT bone densitometer 1003, with their feet secured to wedge-shaped blocks on the platform. The radiation generator is positioned above the lower half of the patient's body. To avoid unnecessary radiation exposure, the patient's upper body is covered with radiation-proof cloth, exposing only the lower body. Figure 1c shows another configuration of the CT bone densitometer 1003, which differs from Figure 1b in that the CT bone densitometer 1003 including the radiation generator is positioned above the patient's upper body. Therefore, when measuring the patient's bone density, it needs to be adjusted to the position of the patient's lower body. It should be noted that in the field of veterinary medicine, the vast majority of research applications use the CT bone densitometer 1003, not the ultrasound bone densitometer 1001.

As shown in Figures 1a-1c, existing bone densitometers not only require operation by professional medical personnel but also have disadvantages such as large size and poor mobility, which prevent their widespread adoption.

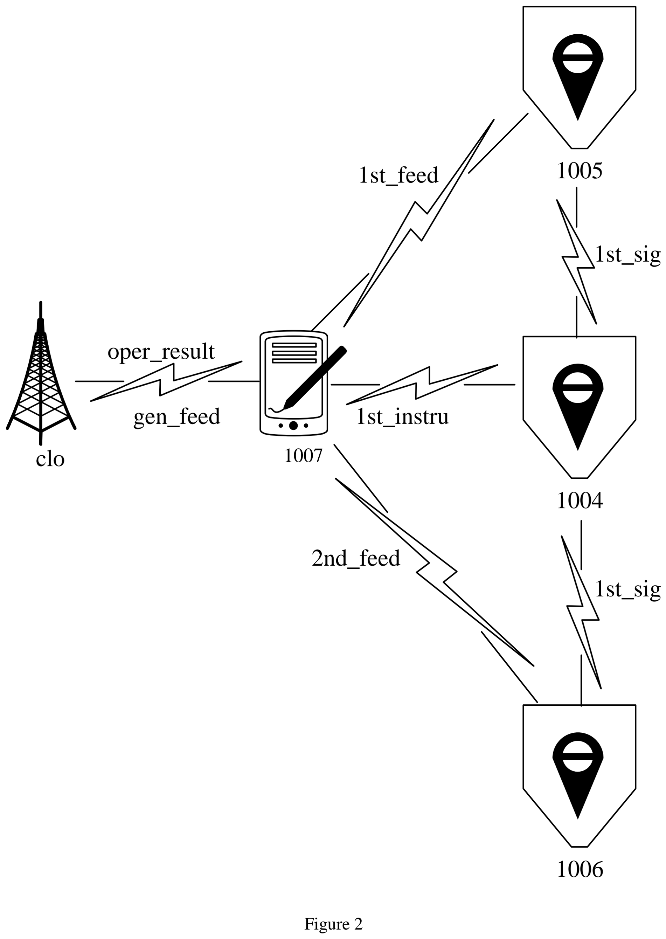

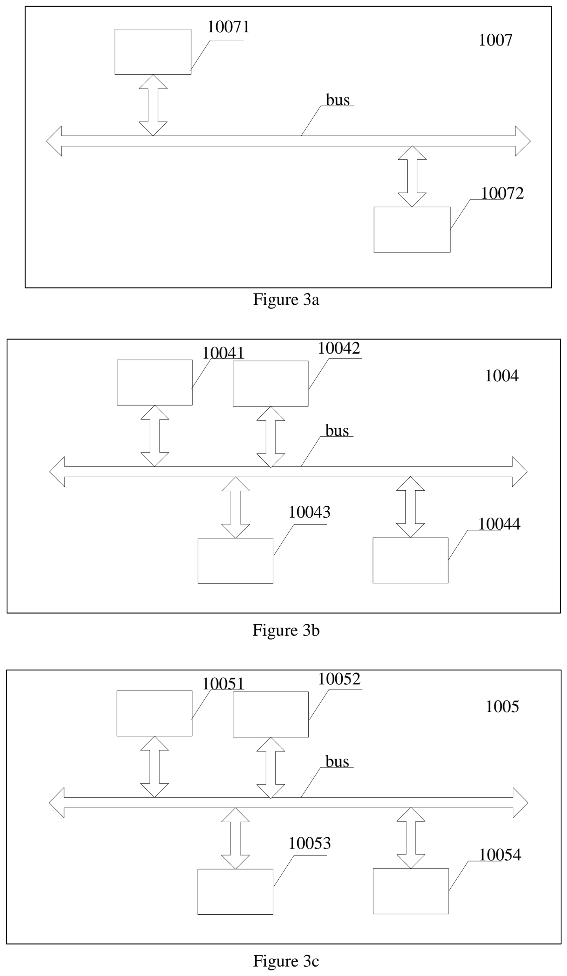

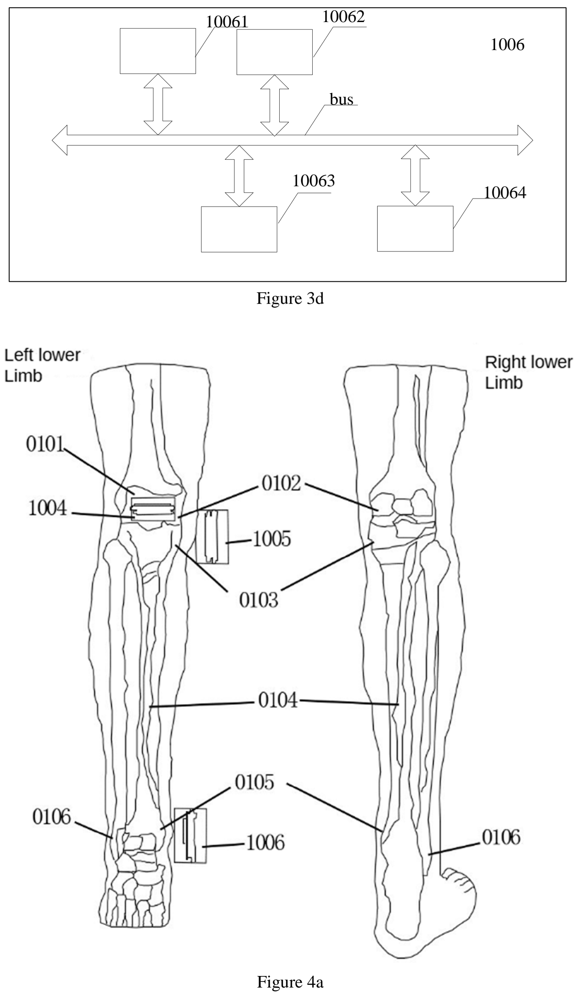

To address at least the aforementioned disadvantages, the present application provides a bone density measurement system. As shown in Figure 2, it includes at least: a first computing device 1007, which is shown in this embodiment as a mobile phone but may take other forms such as a desktop computer or laptop in other embodiments, for purposes such as animal bone age measurement experiments; and at least two signal transceivers - for example, a second transceiver 1005 and a third transceiver 1006. As shown in Figure 3a, the first computing device may include: a first processor 10071, a first non-volatile storage medium 10072 storing a first computer program, wherein the first computing device 1007 issues a first instruction 1st_instru in response to receiving input information.

By way of example, the aforementioned input information may be parameters set by the user of the bone density measurement system for the measured object. For instance, the input information may include: what type of animal the measured object is; whether the current measurement is for primates or humans; the age and gender of the measured object; whether the measured part of the object has ever suffered orthopedic trauma; whether the current measurement requires online or offline mode; whether the current measurement requires precise or rapid measurement; whether specific and accurate bone density results are needed; user feedback on previous bone density results; whether the current measurement requires full automatic mode, offline mode or manual mode; and if offline mode is selected, which specific offline mode is chosen, etc.

In the embodiments of this application, the second transceiver 1005 and third transceiver 1006 are respectively located at different second and third positions, and at a first position different from the second and third positions, there exists a first signal 1st_sig.

The aforementioned first signal 1st_sig can be analogized to ultrasound in prior art, but unlike prior art, this first signal 1st_sig is mechanical vibration perceptible to humans, such as sound audible to humans (20 Hz-20000 Hz). Therefore, compared with prior art bone density measurement devices, the bone density measurement system provided in this application has lower costs and is more conducive to popularization among general public and consumers. For example, the essential ultrasonic transducer chip for medical ultrasound transducers costs at least 3 RMB, while a complete vibration module costs only 2.8 RMB. It is particularly noteworthy that ultrasonic transducer chips themselves cannot emit ultrasound, while a vibration module can emit mechanical vibrations with specific power and frequency. Thus, in practice, the market cost of the bone density measurement system provided in the embodiments of this application is far lower than that of ultrasonic transducers in prior art.

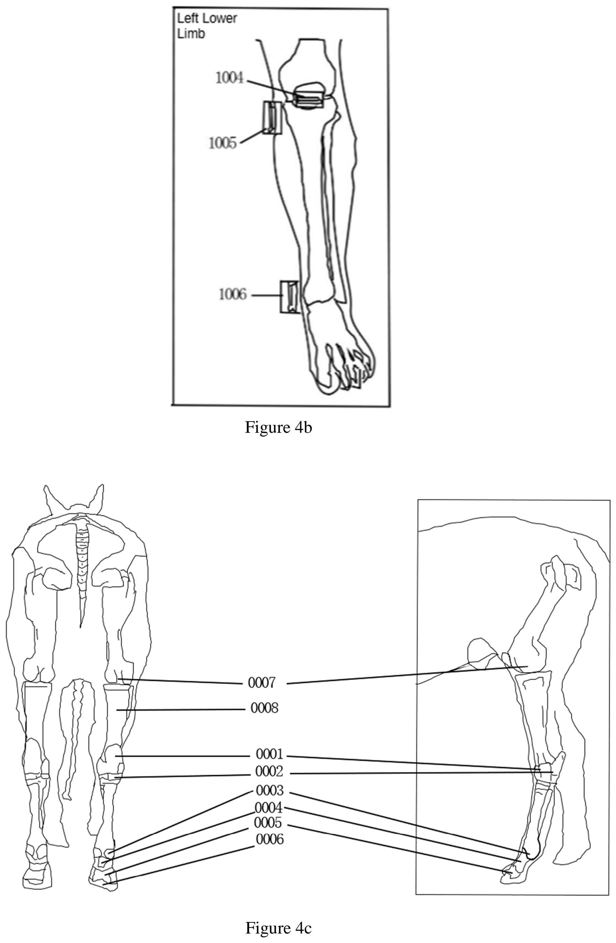

The aforementioned second position and third position are set at different anatomical locations of the measured subject depending on the animal species. The placement principle for these two positions requires that: at these locations, the thickness of soft tissues (e.g., skin, body hair) in animals (including humans) should be minimized to prevent mechanical vibration absorption by soft tissues, which would compromise the final bone density measurement accuracy. For bipedal animals such as primates (including humans), the second position corresponds to the medial malleolus of the tibia 0103 (or alternatively the mid-femoral condyle 0102) of the primate, while the third position is the medial malleolus bone 0105, as illustrated in Figures 4a-4b (these figures depict human lower limbs, though the positioning method remains applicable to primates like chimpanzees). Figure 4a clearly demonstrates the second transceiver 1005 positioned at the transitional zone between the mid-femoral condyle 0102 and tibial medial malleolus 0103, with the third transceiver 1006 located at the medial malleolus bone 0105. Most preferably, both the second transceiver 1005 and third transceiver 1006 are positioned on the same bone; even more preferably, all three transceivers (first transceiver 1004, second transceiver 1005, and third transceiver 1006) are co-located on the same bone. For quadrupedal ungulates, greater positioning flexibility exists due to their more complex limb skeletal structures compared to primates (e.g., cattle, horses, sheep). Referring to Figure 4c, which uses a horse as an example, it illustrates the skeletal structure of a quadrupedal hoofed animal. According to the embodiment corresponding to Figure 4c provided by this application, the second position is approximately at the same horizontal level as the first position (i.e., near the hock 0001 or tarsus 0002), and the third position is at the fetlock 0003 or pastern or coronet 0005 or hoof 0006 of the quadrupedal hoofed animal. Due to the various possible arrangements of the second and third positions on the quadrupedal hoofed animal, it is particularly preferred that the second and third positions can also be located on the same bone. Further preferably, the first transceiver 1004, the second transceiver 1005, and the third transceiver 1006 are arranged on the same bone. Therefore, in Figure 4c, the various computing devices described in the embodiments of this application are no longer shown.

The applicant hereby declares that in Figures 4a and 4b, the first transceiver 1004, second transceiver 1005, and third transceiver 1006 are shown as being located within the same bone - although the first transceiver 1004 and third transceiver 1006 are shown at the junction of different bones. Those skilled in the art may utilize knowledge from the medical field (particularly anatomy) to identify one or more suitable bones for positioning the bone density measurement system; as for whether the first transceiver 1004, second transceiver 1005 and third transceiver 1006 are placed on the "same bone", those skilled in the art should make this determination based on medical (particularly anatomical) knowledge rather than relying solely on the medical names of the bones where they are located. For example, Figure 4a shows the first transceiver 1004 positioned at the kneecap 0101, the second transceiver 1005 at the mid-femoral condyle 0102 or tibial medial malleolus 0103, and the third transceiver 1006 at the medial malleolus bone 0105 - these computing devices are actually located at different parts of the same bone, though these parts have different medical names. Therefore, when ordinary consumers need to use the bone density measurement system disclosed in these embodiments, they must follow certain rules to successfully obtain data about their bone density parameters. Considering that ordinary consumers are not medical professionals, therefore, preferably, the first computing device 1007 includes a display screen. When the first computing device 1007 is activated, it will correspondingly display relevant instructions (in text, images, videos, or other forms), guiding users of the bone density measurement system to install each computing device of the system at optimal and suitable locations; of course, manufacturers of the bone density measurement system may alternatively choose to include a separate printed manual with the system to explain the operating procedures of the bone density measurement system.

The acoustic technology employed in this bone density measurement system involves sound waves within the human-perceptible frequency range of 20 Hz-20,000 Hz. The fundamental principle is: any object of unknown density (which may contain materials of other densities internally, such as a wooden board of unknown density filled with fluid medium) can be treated as a porous object - sound waves of different frequencies passing through such object will exhibit different energy transmission characteristics; in other words, objects of different densities have distinct acoustic resonance frequencies. If the acoustic resonance frequency of a measured object can be determined during measurement, its apparent/overall density can be derived based on relevant prior knowledge.

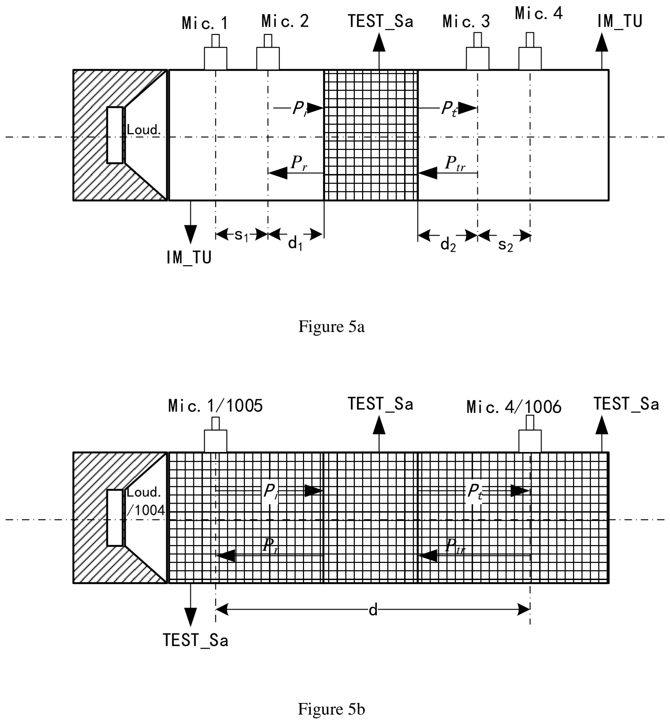

Referring to Figures 5a and 5b, and in conjunction with the literature "Influence of Density on Sound Absorption Coefficient of Fibre Board" (https://doi.org/10.4236/oja.2017.71001; authors: Anand Nandanwar; M. C. Kiran; K. Ch. Varadarajulu), the technical principles of the measurement system provided in the embodiments of this application will be further elaborated below.

Figure 5a is a schematic diagram of measuring the test sample TEST_Sa using the standing wave method. The test sample TEST_Sa (corresponding to the cross-hatched portion) is placed inside the impedance tube IM_TU, with the loudspeaker Loud. serving as the excitation source, and the first microphone Mic.1, second microphone Mic.2, third microphone Mic.3, and fourth microphone Mic.4 serving as vibration/acoustic sensors. Here, the distance between the first microphone Mic.1 and the second microphone Mic.2 is s1, the distance between the second microphone Mic.2 and the test sample TEST_Sa is d1, the distance between the first microphone Mic.1 and the test sample TEST_Sa is s1 + d1, the distance between the third microphone Mic.3 and the fourth microphone Mic.4 is s₂, the distance between the third microphone Mic.3 and the test sample TEST_Sa is d₂, and the distance between the fourth microphone Mic.4 and the test sample TEST_Sa is s₂+ d₂. At the start of the measurement, the loudspeaker Loud. (primarily through the air medium) emits sound waves with power *Pᵢ* and a specific frequency toward the test sample TEST_Sa, a reflected power Pr is generated from the surface of the sample; after passing through the test sample TEST_Sa and reaching the third/fourth microphones Mic.3/4, the power of these sound waves attenuates to *Pₜ*, and reflection generates sound waves with power *Pₜᵣ.* In the measurement scheme shown in Figure 5a, generally, the end of the impedance tube IM_TU (i.e., the far right of Figure 5a or the position indicated by the reference mark "IM_TU") is equipped with a sound-absorbing device (e.g., foam or acoustic rubber), so most of the sound energy from *Pₜ* will be absorbed, causing *Pₜᵣ* to approach zero, and thus the energy represented by *Pₜᵣ* is insufficient to further pass through the test sample TEST_Sa. Therefore, *Pᵣ* mainly consists of the reflected sound energy from the sample surface originating from *Pᵢ*.

Based on the prior art shown in Figure 5a, the embodiments provided in this application are generally based on the technical solution shown in Figure 5b. Compared to the embodiment shown in Figure 5a, in the embodiments of this application, the loudspeaker Loud. is replaced by the first transceiver 1004 or other forms of input energy, and the impedance tube IM_TU is removed (or in other words, the test sample TEST_Sa as a whole replaces the impedance tube IM_TU; or the length of the test sample is extended to the vicinity of the first/fourth microphones Mic.1/4 or the second/third transceivers 1005/1006). In the embodiment shown in Figure 5b, since the sound waves propagate from the excitation source (the loudspeaker Loud. and the first transceiver 1004) to the sensors (the first microphone Mic.1, the fourth microphone Mic.4/the second transceiver 1005, the third transceiver 1006) primarily through the test sample TEST_Sa, *Pₜᵣ* and P, can be neglected. Compared to the embodiment shown in Figure 5a, in the embodiment shown in Figure 5b, the third microphone Mic.3 and the second microphone Mic.2 are eliminated.

The bone density measurement system presented in the embodiments of this application can operate in full-automatic mode (also called "online mode") and offline mode. The various embodiments provided by this application will be further described in detail below with reference to the accompanying drawings.

### Full-automatic mode

In full-automatic mode, the bone density measurement system described in the embodiments of this application requires support from network services provided by telecommunications operators. In this mode, the bone density measurement system described in the embodiments of this application can provide measurement results with the highest accuracy. In Figure 2, the data link between the cloud clo and the first computing device 1007 needs to be provided by a telecommunications operator.

In this mode, the first signal 1st_sig serving as the excitation source of the detection signal needs to be emitted by the dedicated device first transceiver 1004 to ensure consistency, comparability and stability of the detection signal.

In the embodiments of this application, the first transceiver 1004 is set at a first position, which is different from both the second position and third position, but is approximately at the same horizontal level as the second position (when the animal is in a standing state).

The setting of this first position follows these principles: (1) At this first position, the thickness of soft tissues (e.g., skin, body hair) of animals (including humans) should be as small as possible to avoid absorption of mechanical vibrations by soft tissues, which would affect the final bone density measurement results (for example, for primates, setting at the kneecap 0101 is preferred; for quadrupedal ungulates, setting at the hock 0001 or tarsal bone 0002 is preferred). (2) The distance between the first position and second position should be as short as possible, preferably on the same bone. However, considering that air-conducted vibrations may interfere with measurement results, if being on the same bone would make the distance between first and second positions too small, the second position can be set near the first position, but it should be ensured that the first position and the second position are preferably connected through as few bones as possible (most preferably, both first and second positions are set on the same bone). (3) The connection between first position and third position preferably passes through as few bones as possible (because the junctions between different bones cause loss of vibration wave energy, which would adversely affect bone density measurement), but the distance between these two positions should be as long as possible (this is because vibrations can also transmit through air; if the distance is sufficiently long, the influence of air-transmitted vibrations can be excluded while allowing vibrations to pass through as much bone tissue as possible, making the finally measured bone density more representative and eliminating the impact of excessively high or low local bone density).

In full-automatic mode, the working process of the bone density measurement system provided by the embodiments of this application is as follows:
A. The user inputs relevant input information through the first computing device to activate all computing devices, causing them to wake up from sleep mode (optional);
B. After being activated, the first computing device 1007 sends the first instruction 1st_instru to the first transceiver 1004; specifically, the first instruction 1st_instru can be received by the first signal transceiver device 10041 of the first transceiver 1004. When the first signal transceiver device 10041 receives this first instruction 1st_instru, the second processor 10042 of the first transceiver 1004 will accordingly call up the second computer program stored in its second non-volatile storage medium 10043. After this second computer program is executed, the second processor 10042 will activate the vibration generator 10044 to emit the first signal 1st_sig. The first signal 1st_sig, exemplarily, is in the form of mechanical vibration and is a time-domain pulse signal (that is, in the time domain, it can be modeled as an impulse function, where the impulse function has a value of 1 at all frequency points in the frequency domain; moreover, to ensure comparability of measurement data between bone density measurement systems produced by different manufacturers, in the embodiments of this application, the power of the first signal emitted by the first computing device 1007 is limited to 1 watt; if those skilled in the art consider it necessary to increase the power of the first signal to *Pₓ* watts, then normalization operations need to be performed on the subsequent feedback signal amplitudes, for example, the amplitudes of the first and second feedback signals should be correspondingly divided by *Pₓ*). The specific parameters of the first signal 1st_sig can be stored, for example, in the second computer program, or can be stored in the first instruction 1st_instru and then written by the second processor 10042 into the second computer program or related programs inside the second non-volatile storage medium 10043 for execution.
C. The first signal 1st_sig is primarily conducted through the animal's bones and received by the second transceiver 1005 and third transceiver 1006 (specifically, for example, through the second signal transceiver device 10051 of the second transceiver 1005 and the third signal transceiver device 10061 of the third transceiver 1006). After receiving and converting the first signal 1st_sig into electrical signals via the first vibration sensor 10054/second vibration sensor 10064, the third processor 10052 of the second transceiver 1005 calls the third computer program, whose execution enables the third processor 10052 to, for example, activate the second signal transceiver device 10051 to send the first feedback signal 1st_feed to the first computing device 1007. The first feedback signal 1st_feed is a time-domain signal equal to the ratio between (a) the signal parameters (amplitude, phase, etc.) of the first signal 1st_sig as sensed at the second signal transceiver device 10051 after transmission and (b) the distance between the first computing device 1007 and second transceiver 1005. Simultaneously, the fourth processor 10062 of the third transceiver 1006 calls the fourth computer program, whose execution enables the fourth processor 10062 to, for example, activate the third signal transceiver device 10061 to send the second feedback signal 2nd_feed to the first computing device 1007. The second feedback signal 2nd_feed is a time-domain signal whose parameters equal the ratio between (a) the signal parameters (amplitude, phase, etc.) of the first signal 1st_sig as sensed at the third signal transceiver device 10061 after transmission and (b) the distance between the first computing device 1007 and third transceiver 1006.
D. Upon receiving both the first feedback signal 1st_feed and the second feedback signal 2nd_feed, the first processor 10071 of the first computing device 1007 calls the first computer program to derive the operation result oper_result (i.e., the measured object's bone density) based on the first feedback signal 1st_feed and second feedback signal 2nd_feed. The execution of the first computer program enables the first processor 10071 to perform at least:
   D.1 The first feedback signal 1st_feed and the second feedback signal 2nd_feed are subtracted in the frequency domain, and the resulting difference is divided by the amplitude of the first feedback signal to obtain a third feedback signal 3rd_feed, expressed as |3rd_feed|=|1st_feed (frequency domain) - 2nd_feed (frequency domain) |/| 1st_feed(time domain or frequency domain)|, where "|*x*|" denotes taking the absolute value of "x" (alternatively, one may first subtract the first feedback signal 1st_feed from the second feedback signal 2nd_feed, then divide the difference by the amplitude of the first feedback signal 1st_feed, and subsequently apply Fast Fourier Transform (FFT) to the resulting value to obtain the third feedback signal 3rd_feed; theoretically, if the distance between the second transceiver 1005 and the first transceiver 1004 is sufficiently small, the amplitude of the first feedback signal |1st_feed(time domain or frequency domain)| equals the power of the first signal and equals 1 watt, allowing the above equation to be simplified to |3rd_feed| = |1st_feed(frequency domain) - 2nd_feed(frequency domain)|); the value range of the third feedback signal 3rd_feed is defined as: x₃={*x*₃₋₁, *x*₃₋₂, *...x*₃₋ₙ}, where n may correspond to either the number of FFT points when converting the time-domain first feedback signal 1st_feed and second feedback signal 2nd_feed into frequency-domain signals, or the number of FFT points for the aforementioned difference (time domain). Here, *x*₃₋₁ represents the starting frequency point, *x*₃₋ₙ represents the ending frequency point, and in the embodiments of this application, the frequency step size is fixed and can be numerically calculated as |*x*₃₋ₙ - *x*₃₋₍ₙ₋₁₎|. Accordingly, its range of values is defined as {*y*₃₋₁, *y*₃₋₂, ...*y*₃₋ₙ}, where *y*₃₋ₙ represents the amount of energy attenuation at frequency point n;

Wherein, considering that the third feedback signal 3rd_feed may have multiple samples within a certain time period, that is, the measurement activity was performed multiple times (assumed to be "m" times) during this period, or alternatively, there are multiple first signals 1st_sig (correspondingly, there are also multiple first feedback signals 1st feed and second feedback signals 2nd_feed, totaling "m"). Therefore, these measurement data can be arranged in a matrix Y3: $Y 3 = \left[\begin{matrix}{y}_{3 - 1 - 1} & {y}_{3 - 1 - 2} & ⋯ & {y}_{3 - 1 - n} \\ {y}_{3 - 2 - 1} & {y}_{3 - 2 - 2} & ⋯ & {y}_{3 - 2 - n} \\ ⋮ & ⋮ & ⋮ & ⋮ \\ {y}_{3 - m - 1} & {y}_{3 - m - 1} & ⋯ & {y}_{3 - m - n}\end{matrix}\right]$

Corresponding approximately to the frequency range of the first signal 1st_sig, the frequency range of the third feedback signal 3rd_feed is 2000 Hz-7000 Hz, with the number of frequency points being one of the following: 16, 32, 64, 128, 256, 512, 1024, 2048. However, it should be specially noted that to satisfy the requirements of the Nyquist sampling theorem, the sampling frequency (and/or number of frequency points) of the first signal 1st_sig should be at least twice that of the third feedback signal 3rd_feed (preferably five times the sampling frequency and/or number of frequency points of the third feedback signal 3rd_feed).

D.2 Perform principal component analysis (PCA) on the matrix Y3 formed by the third feedback signal 3rd_feed to obtain a set of key i critical frequency points *x*={*x* _{key 1}, *x* _{key 2},...*x* _{key i}}, and the signal values y = {*ŷ*_{key 1}, *ŷ*_{key 2} ... *ŷ*_{key i}} corresponding to each frequency point in the set x (where ${\hat{y}}_{key i} = {\sum }_{q = 1}^{m} {y}_{3 - q - key i} / m$ represents: the arithmetic mean of all observations at a given frequency *x* _{key i}, or it also represents: the arithmetic mean of all elements in the column of matrix Y3 where the frequency*x* _{key i} is located. For example: if the number of observations/measurements is 4 and x includes the frequency point 500 Hz, then the arithmetic mean of all matrix elements in the column where the frequency point 500 Hz is located in matrix Y3 is used to solve for the corresponding *ŷ*_{key i}). The eigenvalues λ={*λ*₁, *λ*₂......*λ*ᵢ} corresponding to each frequency point in the set x at each frequency are also obtained. To save computational resources, the embodiments of this application do not consider frequency points with an eigenvalue of 0, meaning that in the embodiments of this application, all eigenvalues are greater than 0. Here, i≤n. PCA has been widely used in academic fields, and all the PCA-related steps described above can be implemented (for specific examples, see "Research on Urban Integration at County Level - Taking the Southeast Sector of Zhaoqing City, Guangdong Province of China as an Example" by Li Jianxiong published in Issue 1, 2021 of Economist; furthermore, Li Jianxiong's research shows that the sum of the energies of the two largest eigenvalues accounts for more than 95% of the total eigenvalue energy, therefore in the preferred embodiments of this application, only two eigenvalues are considered, while the remaining eigenvalues are set to zero). Preferably, PCA is performed in the cloud clo to avoid consuming the computational resources of the first computing device 1007 as a local device. However, if the computational resources of the first computing device 1007 are sufficiently large, for example, if the first computing device 1007 itself is a dedicated server, PCA can also be executed at the first computing device 1007.

It should be specifically explained that since PCA needs to be performed on Y3, and the PCA process involves eigenvalue decomposition, it is preferable that the number of rows in Y3 should equal the number of columns (i.e., m=n); if it is indeed impossible to conduct multiple observations in the time domain (i.e., m=n cannot be achieved; or the number of observation activities m in the time domain cannot reach the number of FFT/IFFT points n), the minimum requirement is that m should not be less than half of n, meaning the number of observed samples in the time domain should at least reach half of the FFT points.

Input the set x into the first database to find the corresponding frequency response {*ẏ*_{key 1}, *ẏ*_{key 2} ... *ẏ*_{key i}} at each frequency point in the set x. This frequency response is stored in the first database, which is constructed as follows: time pulse vibrations as described earlier are emitted on animals with known bone density and specific biological attributes (e.g., gender, species, age, etc.). By monitoring the energy difference in the frequency domain of feedback signals at unit distances between different second and third positions, the monitored frequency response *ẏ* = {*ẏ*₃₋₁, *ẏ*₃₋₂ ... *ẏ*₃₋ₙ} at frequencies x₃={*x*₃₋₁, *x*₃₋₂, ...*x*_{3-*n*}} is obtained. It should be noted that since bone density measurements are required for multiple animals of the same species with different bone densities, the same frequency point *x*₃₋ₙ for the same species may correspond to different frequency responses *ẏ*₃₋ₙ. In this case, one *x* _{key i} may correspond to multiple values _{key i}.

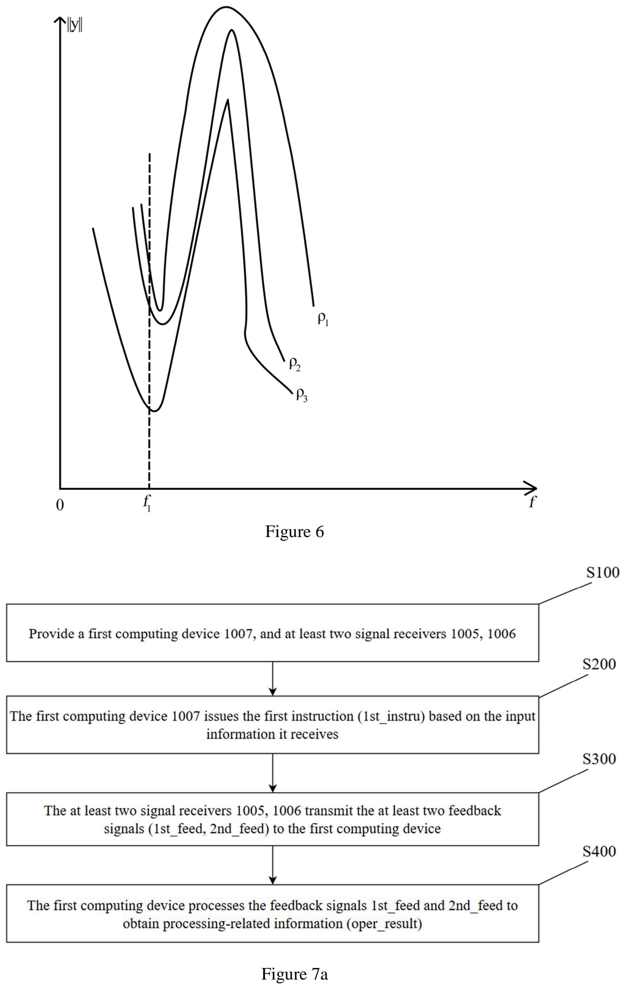

For example, referring to Figure 6, it shows the frequency response ∥y∥ (vertical axis) of a certain animal with different bone densities ρ1, ρ2, ρ3 at various sound frequencies *f* (horizontal axis). It can be observed that at any given frequency point *f*₁, the frequency responses of bones with different densities are distinct from each other.

It should be noted that the frequency response ∥y∥ is a dimensionless physical quantity because, in the calculation process of the third feedback signal, the difference between the first feedback signal and the second feedback signal must also be divided by the power value (or energy value) of the first feedback signal; moreover, according to the law of energy conservation, it can also be inferred that the lower limit of the frequency response ∥y∥ is 0 and the upper limit is 1. This is because the energy value (power value) of the first feedback signal is close to that of the first signal, but when the first signal is transmitted to the third position, since the distance between the first position and the third position is much greater than that between the first position and the second position, the energy value (power value) of the first signal will undergo a certain degree of attenuation. In other words, theoretically, the amplitude of the first feedback signal should be greater than that of the second feedback signal, so the third feedback signal should generally be less than 1.

Additionally, it should be clarified that although the energy value (power value) of the first feedback signal is close to that of the first signal, during actual measurements, the energy value (power value) of the first feedback signal may exceed that of the first signal. This occurs because the biological organism itself exhibits inherent vibrations, and the measurement process may be subject to interference from external mechanical vibrations/acoustic waves. Therefore, instances where the energy value (power value) of the first feedback signal exceeds that of the first signal represent normal conditions. Consequently, the applicant recommends strictly distinguishing between the energy values (power values) of the first feedback signal and the first signal and advises against casually treating these as numerically approximate or identical physical quantities.

To obtain appropriate bone density data, one embodiment of this application performs the following operation: in oper_result, In each *ẏ*_{key i} of oper_result, find the *ẏ*_{key i} closest to *ŷ*_{key i}, and use the *ẏ*_{key i} closest to *ŷ*_{key i}. as the bone density measurement result corresponding to the frequency point *x* _{key i}. It is particularly important to note that in the embodiments of this application, the process of training the first database using machine learning methods is also involved. During this training process, based on the benchmark bone density data manually entered by technicians from actual measurements, techniques such as neural networks and supervised learning are used to learn the bone density corresponding to different frequency responses at each frequency point. Therefore, the *ẏ*_{key i} closest to *ŷ*_{key i} mentioned above does not necessarily refer to observational data obtained through physiological experiments; it can also be predictive data obtained through machine learning and other technical means.

In another alternative embodiment of the present application, the following operation is performed: In each *ẏ*_{key i} of oper_result, find several *ẏ*_{key i} closest to *ŷ*_{key i} , and apply interpolation/extrapolation algorithms to these *ẏ*_{key i}. The resulting value is the bone density measurement result corresponding to the frequency point *x* _{key i}. For example, select the two *ẏ*_{key i-1} = 1 and *ẏ*_{key i-2} = 1.1 closest to *ŷ*_{key i} = 1.2 (in conjunction with the example in Figure 6, this example can be modeled as: knowing the frequency responses of ρ2 and ρ3 at a certain frequency point *f*₁, solving for ρ1 based on the frequency point *f*₁ and the observations at that frequency point). The bone density *ρ̇*_{key i-1} corresponding to the distribution curve where *ẏ*_{key i-1} is located is 1.02, and the bone density *ρ̇*_{key i-2} corresponding to the distribution curve where *ẏ*_{key i-2} is located is 1.06. Then, through a linear extrapolation algorithm, the estimated bone density value corresponding to *ŷ*_{key i} can be, for example, estimated as: (ρ̇_{key i-2} - ρ̇_{rey i-1})/ (ẏ_{key i-2} - ẏ_{key i-1}) × (ŷ_{key i} - ẏ_{key i-2}) + ρ̇_{key i-2} = (1.06 - 1.02)/(1.1 - 1) × (1.2 - 1.1) + 1.06 = 1.1. For example, select the two = 1 and *ẏ*_{key i-2} = 1.1 closest to *ŷ*_{key i} = 1.03 (in conjunction with the example in Figure 6, this example can be modeled as: knowing the frequency responses of ρ1 and ρ3 at a certain frequency point *f*₁, solving for ρ2 based on the frequency point *f*₁ and the observations at that frequency point). The bone density *ρ̇*_{key i-1} corresponding to the distribution curve where is located is 1.03, and the bone density *ρ̇*_{key i-2} corresponding to the distribution curve where *ẏ*_{key i-2} is located is 1.09. Then, through a linear interpolation algorithm, the estimated bone density value corresponding to *ŷ*_{key i} can be, for example, estimated as: (ρ̇_{key i-2} - ρ̇_{key i-1})/(ẏ_{key i-2} - ẏ_{key i-1}) × (ŷ_{key i} - ẏ_{key i-1}) + *ρ̇*_{key i-1} = (1.09 - 1.03)/(1.1 - 1) × (1.03 - 1) + 1.03 = 1.048.

It should be particularly noted that before the aforementioned matching process occurs, there is also a process of selecting a sample set, where the biological attributes of this sample set, among all samples stored in the first database, are closest to those of the animal body to be measured. The reference data involved in the aforementioned matching process is limited to this sample set. Only under the premise of having identical biological attributes will the frequency response curves of various reference data be parallel to each other.

Preferably, the first database is placed in the cloud clo to save storage resources and computing resources of the first computing device 1007. However, it may also be placed in the first computing device 1007 (if conditions permit).

D.4 After going through the above steps, the operation result oper_result of the first computer program can be obtained (specifically, the bone density measurement results at each characteristic frequency point). In the embodiment shown in Figure 2, the operating result oper_result of the first computer program is fed back from the cloud clo to the first computing device 1007 in the form of a general feedback signal gen_feed. However, as described in item D.3, there are multiple bone density measurement results at this time, and since these bone density results correspond to different frequency points, these bone density measurement results are not necessarily the same. Therefore, in the embodiments of this application, the operating result oper_result (and/or general feedback signal gen_feed) can include bone density measurement results at various characteristic frequency points. Optionally, the operating result oper_result (and/or general feedback signal gen_feed) can also include the reliability of the bone density measurement result at a certain characteristic frequency point. This reliability is calculated as follows: Reliability of bone density at a certain frequency point = (Eigenvalue of bone density at that frequency point / Sum of all eigenvalues) × 100%. After obtaining the bone density and reliability at each characteristic frequency point, a first comprehensive bone density can be calculated accordingly for reference by users of the embodiments of this application. This first comprehensive bone density is calculated as follows:First comprehensive bone density = Σ (Bone density measured at a certain characteristic frequency point × Reliability of bone density at that characteristic frequency point). All bone densities mentioned above, including the bone density calculated at each characteristic frequency point, reliability, and first comprehensive bone density, can be part of the operating result oper_result (and/or general feedback signal gen_feed) and fed back to the user. This feedback can be presented to the user through text and graphical information on the display screen of the first computing device 1007, or through sound signals via the speaker of the first computing device 1007.

The full-automatic mode of the embodiments of this application possesses numerous advantages: (1) The computational and storage resources of the first computing device 1007 as the local terminal can be significantly simplified; (2) The database, particularly the first database, can be shared by different users, and if users subsequently employ CT/ultrasound bone densitometers to conduct precise mapping of the measured object's bone density, the bone density database stored in the database of this application's embodiments can be further updated to provide more accurate reference data for other users or for the same user's subsequent measurements (of course, this requires obtaining user authorization and complying with various regional/national regulations concerning personal information, private data, privacy, etc.); (3) The application of PCA technology reduces the volume of data requiring processing/computation. As technicians familiar with FFT/IFFT understand, the data volume involved in FFT/IFFT solutions is generally large because the number of FFT/IFFT points is typically a positive integer power of 2, and accordingly the data points to be processed also follow this pattern. For example, in the embodiments of this application when applied to humans, the characteristic frequency points generally fall within 2000 Hz-7000 Hz. Even with a frequency step of 100 Hz, this requires processing 50 data points. While adopting larger frequency steps could reduce data volume and computational costs, it would compromise the resolution of final output data. PCA technology reduces the number of frequency points requiring processing to generally fewer than 5, and these characteristic frequency points are selected from larger sample populations, ensuring full representativeness while maintaining sufficient resolution in final output data; (4) PCA technology fully accounts for physiological differences among measured individuals. For instance, although in the applicant's experiments the characteristic frequency points for human applications generally ranged between 2000 Hz-7000 Hz, significant variations exist between individuals - some subjects showed maximum eigenvalue frequency points differing by at least 3000 Hz, while minimum eigenvalue frequency points might differ by less than 50 Hz. That is to say, each measured individual has its own characteristic frequency points. After applying PCA technology, the differences of each individual are fully considered, which is more conducive to obtaining accurate bone density measurement results.

### Offline Mode

In the embodiments provided by the present application, there are also provided technical solutions capable of autonomously measuring bone density locally without relying on external communication/telecommunication services, and/or technical solutions that, while utilizing external communication/telecommunication services, have their computational load significantly compressed. Regarding the "offline mode", the embodiments of the present application describe first/second/third offline modes.

### First Offline Mode

In one embodiment of this application, to avoid the substantial computational demands imposed by PCA and the extended measurement time resulting from multiple emissions of the first signal, the PCA step from the full-automatic mode is omitted. In this case, there is no need to perform multiple measurements within a specific time period, or in other words, even if m>1 as described above, m does not strictly need to satisfy the constraint relationship between m and n outlined in item D.2.

In this first offline mode, if the first transceiver 1004 is present, the technical solutions defined by items A to D.1 in the aforementioned fully automatic mode, as well as the technical solutions associated with items A to D.1, remain applicable to this mode.

After obtaining the third feedback signal 3rd_feed, in this embodiment, the signal is input into a second database (generally, the second database can be locally stored in device 1007). This second database matches the stored reference data one-by-one with the third feedback signal 3rd_feed according to the least squares criterion, selects the reference data with the lowest mean square error relative to 3rd_feed as the bone density, and outputs this bone density as the operation result oper_result of the first computer program.

In other words, assuming the second database contains N (where "N" is not equal to the aforementioned "n") frequency-dependent reference bone density curves ρ₁, ρ₂......ρ_{N}, for the third feedback signal 3rd_feed of length n obtained in the m-th observation (whose mathematical form corresponds to the last row of matrix Y3 in the foregoing description), select from these N reference bone density curves a curve ρₓ such that the mean square error MSE_{ρx-3rd_feed} between ρₓ and 3rd_feed obtained in the m-th observation is the smallest among the mean square errors MSE_{prandom-3rd_feed} between any arbitrary reference bone density curve ρ_{random} and 3rd_feed (where ρₓ is a subset of ρ_{random}). Alternatively, |ρₓ-3rd_feed|²≤|ρ_{random}-3rd_feed|², where ρₓ is a subset of ρ_{random}. It should be noted that, as mentioned above, FFT/IFFT involves a large number of data points. Therefore, if directly comparing the frequency points and corresponding frequency responses of the original 3rd_feed signal with each reference bone density curve, the computational load would still be substantial. Consequently, one may select only certain fixed frequency points for matching according to the least squares criterion. For example, illustratively, if the frequency range of 3rd_feed is 2000-7000 Hz with a step size of 50 Hz, to save computation time for the least squares method, one may consider only the frequency points {2000 Hz, 3000 Hz, 4000 Hz, 5000 Hz, 6000 Hz, 7000 Hz} and their corresponding frequency responses, thereby reducing computational complexity.

Similar to the full-automatic mode described above, this mode may yield multiple bone density measurement results. However, what differs is that these multiple bone density measurement results are obtained through multiple measurement activities conducted at different times, and the bone density measurement results acquired at different times exhibit high correlation. In this mode, the operation result oper_result may include bone density measurement results obtained at different times, as well as a second comprehensive bone density whose value equals the average of the individual bone density measurement results obtained at these different times. In this mode, the second database is built into the local terminal 1007 and does not require additional communication services from telecom providers, enabling users of the bone density measurement system according to these embodiments to utilize the system without network services. Of course, this mode does not exclude the application of the cloud clo, so the aforementioned least squares method for obtaining bone density can also be applied to the cloud clo. Correspondingly, the first database mentioned earlier can serve as the second database, and these bone density measurement results may also be included in the general feedback signal gen_feed from the cloud clo as described above.

### Second Offline Mode

In the second offline mode (also referred to as the "manual mode"), the aforementioned first transceiver does not exist. The first signal is a random knock in any form. For example, the first signal may be generated in the form of a user knocking on the patella using the bone density measurement system described in the embodiments of the present application. In this mode, a calibration procedure also needs to be set in advance to accommodate differences caused by different first signals (for example, the first signal generated by directly knocking with a finger joint and the first signal generated by using a hammer for knocking bones in the orthopedic field obviously differ in amplitude and phase).The calibration step is: (1) if the first computing device 1007 receives input information indicating that the user has selected the manual mode, the second transceiver 1005 is actuated and issues a corresponding prompt to the user (for example, a prompt in the form of an image, flash, or sound), so that the user emits a leading signal/probing signal of the first signal; (2) the second transceiver 1005 monitors the leading signal/probing signal and provides feedback to the first computing device 1007; (3) based on the feedback result from the second transceiver 1005, a threshold range is determined. The threshold range may be determined based on the energy intensity E of the leading signal/probing signal monitored by the second transceiver 1005, for example, the threshold range may be determined as 0.9E-1.1E (i.e., fluctuating ±10% based on E), or may be determined as (1-R%)E-(1+R%)E (i.e., fluctuating ±R% based on E, where 0≤R≤100); (4) a first instruction 1st_instru is issued to instruct the user to formally emit the first signal 1st_sig, and the first instruction 1st instru may be, for example, a prompt in the form of an image, flash, or sound, so that the user can emit the first signal 1st_sig several times in a specific rhythm (for example, instructing the user to knock on the first position of the object to be measured); (5) the several first signals 1st_sig are processed according to the technical solutions described in the fully automatic mode or the first offline mode or the third offline mode (to be described below), with the differences being: (Difference 1) in response to the energy of the first feedback signal 1st_feed in the time domain not falling within the amplitude range, the first computing device 1007 ignores the first feedback signal 1st_feed and the corresponding second feedback signal 2nd_feed; (Difference 2) since the first computing device 1007 ignores specific first signals 1st_sig, this leads to a reduction in the number of measured samples, and therefore, optionally, the first computing device 1007 may also issue a prompt in the form of an image, flash, or sound to instruct the user to continue increasing the number of first signals 1st_sig until the number of first signals 1st_sig meets the required amount, and only then further calculate the bone density data oper_result based on the first signals 1st_sig and the associated third feedback signals 3rd_feed; (Difference 3) as mentioned above, the upper limit of the bone density curve stored in the first/second database as reference data is 1 and the lower limit is 0, and in order to ensure that the third feedback signal 3rd_feed obtained in the second offline mode is comparable to the bone density curve stored in the first database as reference data, after taking the difference between the first feedback signal and the second feedback signal, the difference also needs to be divided by the following term: the product of the upper limit value (1+R%)E of the amplitude range and the amplitude of the first feedback signal. (1+R%)E corresponds to a power value of 1 watt of the first signal in the fully automatic mode, and the reason for dividing by the upper limit value (1+R%)E of the amplitude range is that only by dividing by (1+R%)E can it be ensured that any third feedback signal 3rd_feed can fall within the range [0,1], otherwise, some third feedback signals 3rd_feed would be greater than 1, making the measured third feedback signals 3rd_feed incomparable with the bone density curves stored in the first/second database as reference data. The calculation method of the bone density data oper_result is the same as in the fully automatic mode or the first offline mode or the third offline mode to be described below.

### Third Offline Mode

It should be specifically stated that the third offline mode is only applicable to humans, and it does not provide bone density measurement results, but only provides a non-diagnostic quality assessment result of bone density.

The third offline mode is essentially a simplified version of the aforementioned fully automatic mode and the first/second offline modes. Specifically, the reference data in the previously mentioned first database/second database is replaced with data of populations of different age groups having specific T-scores. At this point, all previously mentioned bone density values (bone density calculated at various feature points, first/second integrated bone density, bone density calculated at different time points) will be replaced with T-scores as defined by the World Health Organization. This mode can significantly compress the sample size stored in the first database/second database, because several bone density values corresponding to specific frequency points may correspond to one or more T-scores (even if there are multiple T-scores, their number is still much smaller than the number of bone density curves). At this point, both the first database and the second database can be configured locally on the terminal 1007, enabling the local terminal 1007 to perform the steps of measuring bone density under any of the aforementioned modes (but the final measurement result is a T-score, not a specific bone density value). Therefore, this third offline mode can provide early warning information for users who may suffer from osteoporosis, enabling relevant users to seek medical attention at a medical institution as early as possible after obtaining the relevant measurement results, to further verify whether they suffer from orthopedic diseases such as osteoporosis.

T-scores are already well known in the medical field, and specific reference can be made to the study by Zhang Xiaoqian, Li Na, Gao Hongyu, et al., titled "Study on the Differences and Clinical Significance of T-scores at Different Sites in People Over 50 Years Old," published in Chinese General Practice (2019, 22(27), pp. 3312-3316), which will not be further elaborated herein.

It should be particularly noted that, prior to the matching process described in any of the above offline modes, there is also a process of selecting a sample set, the biological attributes of which are the closest to the biological attributes of the animal body to be measured among all the biological attributes of the samples stored in the first database/second database. The reference data involved in the above-mentioned matching process is limited to this sample set. Only under the premise of having the same biological attributes will the frequency response curves of the respective reference data be parallel to each other.

The first computing device 1007, as described above, may be a smartphone, or may be a laptop or notebook computer. Therefore, in practice, the only components with specialized characteristics that consumers truly need to obtain from the manufacturer are the first transceiver 1004 (preferably equipped), the second transceiver 1005, and the third transceiver 1006. These three components only involve parts that are already widely used in the field of electronics, such as mechanical vibration generators (or sound generators), vibration sensors (or sound sensors), signal transceivers, FFT/IFFT modules, etc. Therefore, compared with ultrasonic bone densitometers and radiation-based bone densitometers disclosed in the prior art, the bone density measurement system provided in the embodiments of the present application has a significant cost advantage; the above-mentioned devices can all be implemented using low-voltage electronic components and are not instruments or devices regulated by national/government authorities, making them suitable for broader public dissemination; furthermore, the bone density measurement system provided in the embodiments of the present application is compact in size and suitable for measuring the bone density of large animals, providing researchers in the field of zoology with a system for measuring the bone density of large animals.

Although the technical solution provided in the embodiments of the present application has several advantages, it should be specifically stated that the technical solution involved in the embodiments of the present application is non-diagnostic in nature, because: (1) First, the bone density measurement system described in the embodiments of the present application is intended to obtain the bone density of the object to be measured. Bone density of a biological body is an objectively existing physical/physiological parameter. By referring to certain bone density classification standards, it can reflect whether the object to be measured may have a specific disease (e.g., osteoporosis), but bone density itself cannot reflect whether the object to be measured is healthy or suffers from a certain disease; (2) As mentioned above, the signals related to the biological body only include the first feedback signal 1st feed, the second feedback signal 2nd_feed, and the third feedback signal 3rd_feed generated from these two signals. None of these signals can directly reflect the bone density of the object to be measured. These signals need to undergo further matching and calculation to finally obtain the operation result oper_result that reflects bone density information. However, even the operation result oper_result is an indirect result, which is an approximate fitting of the bone density of the object to be measured based on existing bone density reference data. Therefore, the technical solution provided in the embodiments of the present application is essentially different from bone densitometers in the prior art that can directly yield bone density data, and any result provided by the technical solution of the present application should not be used as a diagnostic basis for determining the bone condition of a patient in clinical settings; (3) Even ultrasonic bone densitometers in the prior art, which have higher detection accuracy than the bone density measurement system of the present application (and whose measurement results can directly reflect the bone density of the object to be measured), are not used in clinical settings as diagnostic bases for determining the bone condition of a patient. Only radiation-based bone densitometers can be used as diagnostic bases for determining the bone condition of a patient (Xu Shengkang, "Bone Density Examination? Which is the Most Reliable?", June 15, 2024, https://v.douyin.com/iyP9MF17/); (4) Therefore, the various parameters used in the technical solution of the present application can only be regarded as intermediate results for inferring the bone density of an animal body. The direct purpose of applying these parameters is not to obtain diagnostic results or health status, but only to obtain information as intermediate results from a living human or animal body. As of the effective priority date/effective filing date of the present application, based on the medical knowledge in the prior art, it is not possible to directly derive diagnostic results or health status regarding bone condition from the content disclosed in the present application. Therefore, the various parameters involved in the embodiments of the present application should be regarded as intermediate results rather than diagnostic results used as a basis for diagnosing diseases.

Exemplarily, in the embodiments of the present application, the internal components of each computing device (e.g., computing devices 1007/1004/1005/1006), such as processors 10042/10062/10071 and memories 10043/10063/10072, are exemplarily connected via a communication bus. Such circuit connections are known to those skilled in the art, but are not limited thereto, as shown in Figures 3a to 3d.

It is understood that, since system-required signals such as the first instruction 1st_instru, the first feedback signal 1st feed, and the second feedback signal 2nd_feed can be respectively generated by computing devices such as the first computing device 1007, the second transceiver 1005, and the third transceiver 1006, and may be in the form of radio, laser, visible light, Bluetooth, or infrared light, the above-mentioned computing devices may also additionally generate other signals for communication between the above-mentioned computing devices, apart from the system-required signals mentioned above. For example, the second transceiver 1005 and the third transceiver 1006 may be in a sleep state during idle periods, and when the first computing device 1007 operates in response to input information, it may emit a second signal (which may also be in the form of radio, laser, visible light, Bluetooth, or infrared light) to wake up the second transceiver 1005 and the third transceiver 1006.

Each computing device may use the above-mentioned signals to measure the distance between each other, and the above-mentioned distance may be used in the bone density measurement system disclosed in the embodiments of the present application. The bone density measurement system may also be additionally equipped with dedicated devices (e.g., a ruler, by which the user manually measures the distance between the relevant computing devices); for example, the vibration generator 10044 may be a sound vibration generator, and the second transceiver 1005, the third transceiver 1006, the first vibration sensor 10054, and the second vibration sensor 10064 may additionally include sound sensing devices such as microphones for sensing sound waves in the air (the sound waves here preferably refer to sound waves in the frequency range of 20 Hz-20000 Hz perceptible to humans, but may also include sound waves imperceptible to humans). After the sound vibration generator emits sound, it may automatically calculate the distance between the relevant computing devices based on the transmission time of the echo signal fed back by the sound sensing device.

The term "and/or" used in the present application indicates that the relationship between components/steps is "and" or "or", and the components/steps may be absent. For example, "a and/or b" indicates four situations: "a and b", "a or b", "only a", and "only b".

In some non-limiting embodiments or aspects, the processor may be implemented in hardware, software, or a combination of hardware and software. For example, processors 10071/10062/10052/10042 may include processors (e.g., central processing units (CPUs), graphics processing units (GPUs), accelerated processing units (APUs), etc.), microprocessors, digital signal processors (DSPs), and/or any processing components that can be programmed to perform functions (e.g., field-programmable gate arrays (FPGAs), application-specific integrated circuits (ASICs), etc.). Memories 10043/10053/10063/10072 may include random access memory (RAM), read-only memory (ROM), and/or other types of dynamic or static memory (e.g., flash memory, magnetic memory, optical memory, etc.) for storing information and/or instructions for use by processors 10071/10062/10052/10042.

Memories 10043/10053/10063/10072 may store information and/or software associated with the operation and use of the processors. For example, memories 10043/10053/10063/10072 may include hard drives (e.g., magnetic disks, optical disks, magneto-optical disks, solid-state drives, etc.), compact discs (CDs), digital versatile discs (DVDs), floppy disks, cassettes, tapes, and/or other types of computer-readable media, along with corresponding drives. The term "non-volatile" or "non-volatile storage (NVS)" refers to memory of this type: when the computer is turned off or loses its external power supply, the contents stored in the memory can be retained.

Input ports for receiving information may include components that receive user input (e.g., touchscreen displays, keyboards, keypads, mice, buttons, switches, microphones, etc.). Additionally or alternatively, input ports may include sensors for sensing information (e.g., global positioning system (GPS) components, accelerometers, gyroscopes, actuators, etc.). Output ports may include components that provide output information from the device (e.g., displays, speakers, one or more light-emitting diodes (LEDs), etc.).

The computer programs described herein are configured to be executed by hardware machines (such as C, C++, Fortran, Java, Basic, Matlab, etc.) or modeling/simulation programs (such as Simulink, Stateflow, GNU Octave, or LabVIEW MathScript). It is possible to implement modules using physical hardware incorporating discrete or programmable analog, digital, and/or quantum hardware. Examples of programmable hardware include: computers, microcontrollers, microprocessors, application-specific integrated circuits (ASICs); field-programmable gate arrays (FPGAs); and complex programmable logic devices (CPLDs). Computers, microcontrollers, and microprocessors are programmed using languages such as assembly, C, C++, etc. FPGAs, ASICs, and CPLDs are often programmed using hardware description languages (HDLs), such as VHSIC Hardware Description Language (VHDL) or Verilog, which configure the connections between less functional internal hardware modules on programmable devices.

The computing devices described herein may include personal desktop computers, personal notebook computers, servers, smartphones, supercomputers, etc., known in the prior art.

Embodiments of the technology have been described as implemented in circuitry and/or computer-executable instructions. It should be understood that some embodiments may take the form of methods, at least one example of which has been provided. The actions performed as part of the method may be ordered in any suitable way. Accordingly, embodiments may be constructed in which actions are performed in an order different from that illustrated, which may include performing some actions simultaneously, even if shown as sequential actions in illustrative embodiments.

The various aspects of the above-described embodiments may be used individually, in combination, or in arrangements not specifically discussed in the foregoing embodiments, and thus their application is not limited to the details and arrangements of components set forth in the foregoing description or illustrated in the drawings. For example, aspects described in one embodiment may be combined in any way with aspects described in other embodiments.

In the claims, ordinal terms such as "first," "second," "third," etc., used to modify claim elements do not imply any priority, precedence, or order of the claim elements relative to one another or any temporal order of method steps, but are merely used as labels to distinguish one claim element having a certain name from another element having the same name (but using an ordinal term) for the purpose of distinguishing claim elements.

Furthermore, the terminology and phrasing used herein are for the purpose of description and should not be regarded as limiting. As used herein, the terms "comprising," "including," "having," "containing," "involving," and variations thereof are intended to encompass the listed items and their equivalents as well as additional items.

The term "exemplary" as used herein means serving as an example, instance, or illustration. Any embodiment, implementation, process, or feature described herein as exemplary is therefore to be understood as illustrative and not necessarily preferred or advantageous, unless otherwise stated.

Accordingly, several aspects of at least one embodiment have been described, and it should be understood that those skilled in the art will readily conceive of various changes, modifications, and improvements. Such changes, modifications, and improvements are intended to be part of this disclosure and to fall within the spirit and scope of the principles described herein. Accordingly, the foregoing description and drawings are merely illustrative.

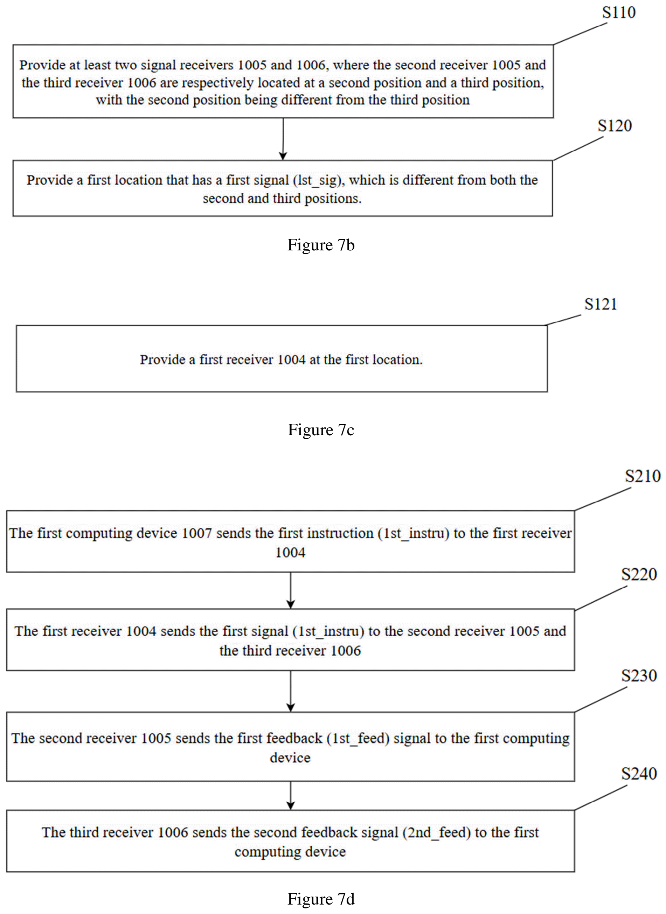

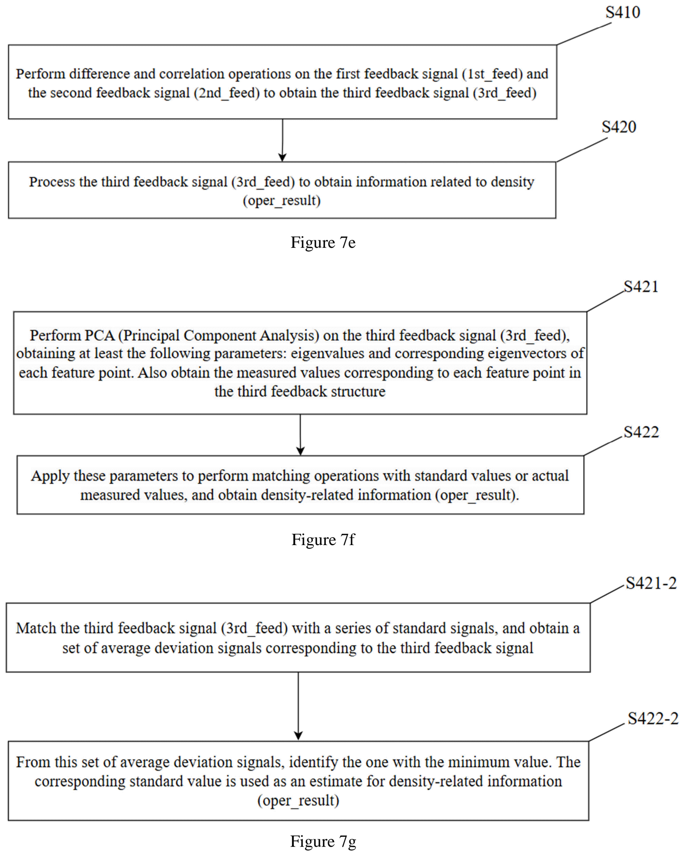

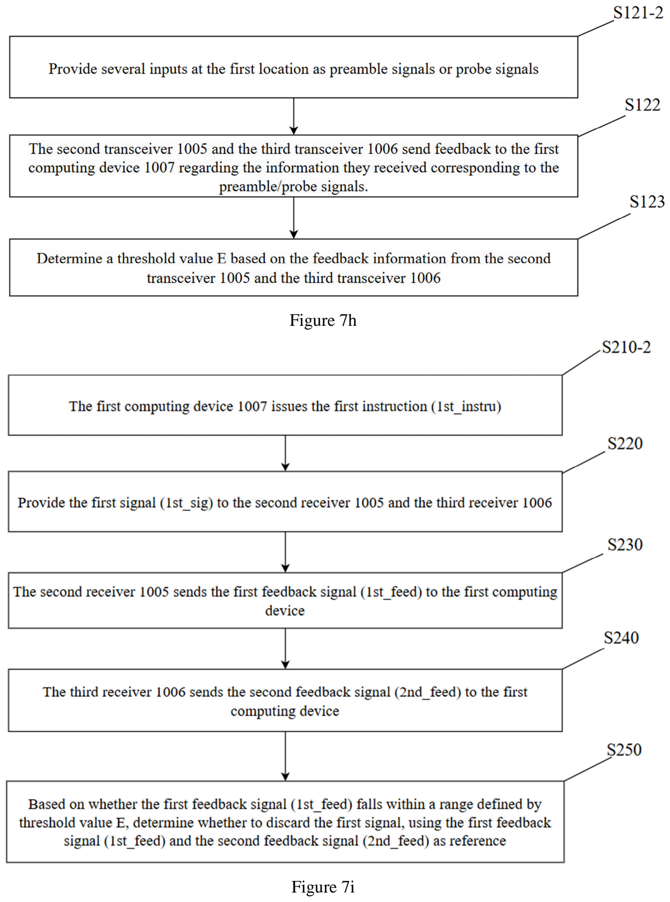

Although the embodiments of the present application are described using different computing devices (e.g., computing devices 1007/1004/1005/1006) as examples, those skilled in the art may implement the technical content described in the embodiments of the present application through different methods, processes, or steps, thereby obtaining various parameters and measurement results already described in the embodiments of the present application.
For example, for non-diagnostic purposes, by executing the method, process, and steps applicable to non-human animals as shown in Figure 7a (specifically, by executing steps S100, S200, S300, S400), the technical objective of the first embodiment of the present application can be achieved;
For example, for non-diagnostic purposes, by executing the method, process, and steps applicable to non-human animals as shown in Figure 7b (specifically, by executing steps S110, S120), the technical objective of the second embodiment of the present application can be achieved;
For example, for non-diagnostic purposes, by executing the method, process, and steps applicable to non-human animals as shown in Figure 7c (specifically, by executing step S121), the technical objectives of the third and fourth embodiments of the present application can be achieved;
For example, for non-diagnostic purposes, by executing the method, process, and steps applicable to non-human animals as shown in Figure 7d (specifically, by executing steps S210, S220, S230, S240), the technical objectives of the fifth and sixth embodiments of the present application can be achieved;
For example, for non-diagnostic purposes, by executing the method, process, and steps applicable to non-human animals as shown in Figure 7e (specifically, by executing steps S410, S420), the technical objective of the seventh embodiment of the present application can be achieved;
For example, for non-diagnostic purposes, by executing the method, process, and steps applicable to non-human animals as shown in Figure 7f (specifically, by executing steps S421, S422), the technical objective of the eighth embodiment of the present application can be achieved; in particular, step S422 may further include the computational processes described in the ninth and tenth embodiments (e.g., interpolation, extrapolation processes) to obtain information related to bone density (e.g., bone density corresponding to several frequency points and/or the first integrated bone density);
For example, for non-diagnostic purposes, by executing the method, process, and steps applicable to non-human animals as shown in Figure 7g (specifically, by executing steps S421-2, S422-2), the technical objective of the eleventh embodiment of the present application, as an alternative embodiment to the eighth embodiment, can be achieved;
For example, for non-diagnostic purposes, by executing the method, process, and steps applicable to non-human animals as shown in Figures 7h and 7i, the technical objectives of the fourteenth and fifteenth embodiments of the present application, as alternative embodiments to the eighth and eleventh embodiments, can be achieved; in particular, steps S121-2, S122, and S123 described in Figure 7h collectively replace step S121 described in Figure 7c, and steps S210-2, S220, S230, S240, and S250 described in Figure 7i replace steps S210, S220, S230, and S240 in Figure 7d. The steps shown in Figures 7h and 7i may also be combined with the steps described in Figures 7a, 7b, 7e, 7f, and 7g.

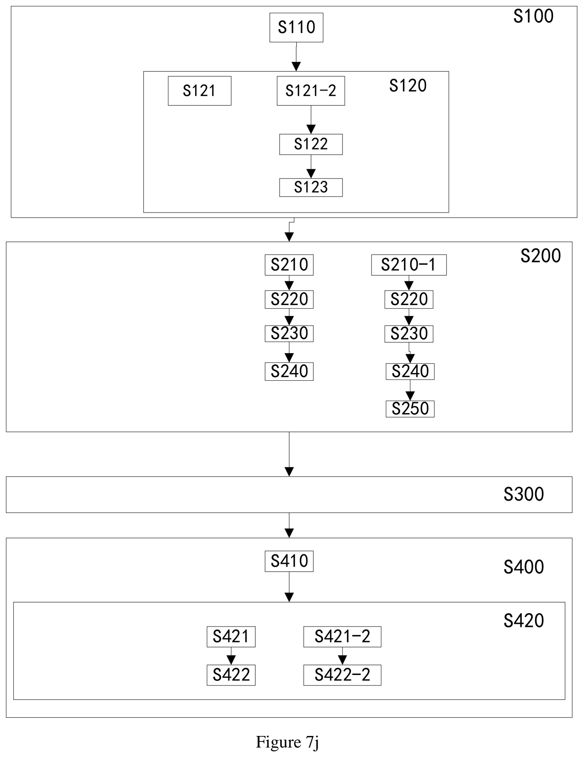

Figure 7j is a generalized view of the various views from Figures 7a to 7i, and the specific content of each step can be found in Figures 7a to 7i.

The methods, processes, and steps described in Figures 7a to 7j may also apply the technical solutions and features described in the twelfth, thirteenth, and twentieth to twenty-first embodiments.

## Claims

1. A bone density measurement system, comprising:
a first computing device (1007), having a first processor (10071) and a first non-volatile storage medium (10072), wherein the first non-volatile storage medium (10072) stores a first computer program, and the first computing device (1007) is further configured to issue a first instruction (1st_instru) in response to receiving input information;
at least two signal transceivers, the at least two signal transceivers comprising a second transceiver (1005) and a third transceiver (1006);
wherein, in response to receiving at least two feedback signals from the second transceiver (1005) and the third transceiver (1006), the first processor (10071) is configured to invoke the first computer program and output an operation result (oper_result) of the first computer program.

2. The bone density measurement system according to claim 1, wherein:
the second transceiver (1005) and the third transceiver (1006) are respectively located at a second position and a third position different from each other, and a first signal (1st_sig) is present at a first position different from the second position and the third position.

3. The bone density measurement system according to claim 2, further comprising a first transceiver (1004), wherein, in response to receiving input information, the first computing device (1007) issues the first instruction (1st_instru) to cause the first transceiver (1004) to emit the first signal (1st_sig).

4. The bone density measurement system according to claim 3, wherein:
the first transceiver (1004), the second transceiver (1005), and the third transceiver (1006) are respectively located at a first position, a second position, and a third position different from each other.

5. The bone density measurement system according to claim 3 or 4, wherein:
the first transceiver (1004) comprises at least a first signal transceiving device (10041), a second processor (10042), a second non-volatile storage medium (10043), and a vibration generator (10044), wherein the second non-volatile storage medium (10043) stores a second computer program;
the second transceiver (1005) comprises at least a second signal transceiving device (10051), a third processor (10052), a third non-volatile storage medium (10053), and a first vibration sensor (10054), wherein the third non-volatile storage medium (10053) stores a third computer program;
the third transceiver (1006) comprises at least a third signal transceiving device (10061), a fourth processor (10062), a fourth non-volatile storage medium (10063), and a second vibration sensor (10064), wherein the fourth non-volatile storage medium (10063) stores a fourth computer program.

6. The bone density measurement system according to claim 5, wherein:
in response to the first signal transceiving device (10041) receiving the first instruction (1st_instru), the second processor (10042) invokes the second computer program to cause the vibration generator (10044) to emit the first signal (1st_sig);
in response to the second signal transceiving device (10051) receiving the first signal (1st_sig), the first vibration sensor (10054) converts the first signal (1st_sig) into an electrical signal for processing by the third processor (10052), and the third processor (10052) invokes the third computer program to cause the second signal transceiving device (10051) to emit a first feedback signal (1st_feed);
in response to the third signal transceiving device (10061) receiving the first signal (1st_sig), the second vibration sensor (10064) converts the first signal (1st_sig) into an electrical signal for processing by the fourth processor (10062), and the fourth processor (10062) invokes the fourth computer program to cause the third signal transceiving device (10061) to emit a second feedback signal (2nd_feed);
in response to the first computing device receiving the first feedback signal (1st_feed) and the second feedback signal (2nd_feed), the first processor (10071) invokes the first computer program to operate on the first feedback signal (1st_feed) and the second feedback signal (2nd_feed) and obtain the operation result (oper_result) of the first computer program;
wherein the at least two feedback signals comprise the first feedback signal (1st_feed) and the second feedback signal (2nd_feed).

7. The bone density measurement system according to claim 6, wherein the operation on the first feedback signal (1st_feed) and the second feedback signal (2nd_feed) comprises:
performing a difference and correlation operation on the first feedback signal (1st_feed) and the second feedback signal (2nd_feed) to obtain a third feedback signal (3rd_feed), wherein the third feedback signal (3rd_feed) is a frequency domain signal;
obtaining information about bone density based on the third feedback signal (3rd_feed).

8. The bone density measurement system according to claim 7, wherein obtaining information about bone density based on the third feedback signal (3rd_feed) comprises:
performing principal component analysis on the third feedback signal (3rd_feed) to obtain at least a plurality of frequency points associated with the third feedback signal (3rd_feed), eigenvalues corresponding to the plurality of frequency points, and a plurality of observation values corresponding to the plurality of frequency points;
wherein the correlation operation at least comprises: further dividing the difference between the first feedback signal and the second feedback signal by the amplitude of the first feedback signal;
inputting the plurality of frequency points, the eigenvalues, and the plurality of observation values into a first database, wherein the first database is configured to perform a matching operation between the plurality of frequency points and reference data stored in the first database and return/output a plurality of pieces of information about bone density.

9. The bone density measurement system according to claim 8, wherein performing the matching operation and returning a plurality of pieces of information about bone density comprises:
for each frequency point among the plurality of frequency points, searching within the first database for data corresponding to the frequency point, selecting the data closest to the observation value of the frequency point as the bone density, and outputting the bone density; or
for each frequency point among the plurality of frequency points, searching within the first database for data corresponding to the frequency point, selecting a plurality of data closest to the observation value of the frequency point, and applying an interpolation/extrapolation algorithm to the plurality of data to obtain an interpolation/extrapolation result, using the interpolation/extrapolation result as the bone density, and outputting the bone density.

10. The bone density measurement system according to claim 9, wherein the operation result (oper_result) of the first computer program comprises the bone density obtained for each frequency point among the plurality of frequency points and/or a first integrated bone density, wherein the first integrated bone density is obtained by performing a weighted operation on the bone density corresponding to each frequency point among the plurality of frequency points, the weighted operation comprising: multiplying the bone density corresponding to each frequency point by a weight corresponding to the eigenvalue of the frequency point based on the proportion of the eigenvalue of each frequency point in the principal component analysis relative to all eigenvalues, and summing the results.
